# EUROPEAN PATENT SPECIFICATION

(11) **EP 2 895 076 B1**
(45) Date of publication and mention of the grant of the patent: **09.09.2020**
(21) Application number: 13799371.3
(22) Date of filing: 12.09.2013
(51) Int. Cl.: A61B 8/00

(54) **ULTRASOUND IMAGING DEVICE OPERATED BY MOBILE DISPLAY DEVICE AND ULTRASOUND IMAGING SYSTEM**
DURCH EINE BEWEGLICHE ANZEIGEVORRICHTUNG BETRIEBENE ULTRASCHALLBILDGEBUNGSVORRICHTUNG UND ULTRASCHALLBILDGEBUNGSSYSTEM
DISPOSITIF D'IMAGERIE ULTRASONIQUE ACTIONNÉ PAR UN DISPOSITIF D'AFFICHAGE MOBILE ET SYSTÈME D'IMAGERIE ULTRASONIQUE

(30) Priority: 13.09.2012 US 201261700416 P
(43) Date of publication of application: 22.07.2015
(73) Proprietor: Koninklijke Philips N.V., 5656 AG Eindhoven (NL)
(72) Inventor: POLAND, McKee Dunn, NL-5656 AE Eindhoven (NL)
(74) Representative: Philips Intellectual Property & Standards
(86) International application number: PCT/IB2013/058495
(87) International publication number: WO 2014/041503

(56) References cited:
- WO-A1-2010/076723
- JP-A- 2003 033 350
- US-A1- 2003 139 664
- US-A1- 2004 179 332
- US-A1- 2008 119 731
- US-A1- 2008 194 951
- US-A1- 2012 054 401
- US-B1- 6 440 072
- US-B1- 6 447 451

## Description

### FIELD OF THE INVENTION

The present invention relates to an ultrasound image acquisition station for use together with a mobile display device to form an ultrasound imaging system. Further, the present invention relates to an ultrasound imaging system for providing an image of area tomographic plane or volume, for example an anatomical view within a body of a patient.

### BACKGROUND OF THE INVENTION

Ultrasound imaging systems are widely known in the art. They are in particular used to provide anatomical imaging of patients. Both two-dimensional and three-dimensional imaging of bodies of patients is known to provide a reliable tool for medical practitioners to view parts of a body of a patient without the need for any surgical steps.

Ultrasound imaging systems are complete stations that may be fixed to a certain location and are often movable on rollers to provide flexible use in different locations. The ultrasound imaging systems provide for every component needed to acquire ultrasound images, i.e. input devices, display devices, any computer hardware needed to run the ultrasound imaging system and the specific software for acquiring, rendering and displaying the ultrasound images. Further, the ultrasound imaging systems comprise at least one probe, carrying one- or two-dimensional transducer arrays to scan the body of a patient either manually or automatically.

Of course, providing fully set up ultrasound imaging systems comprising every component as mentioned above makes these systems not only relatively costly but also large, heavy and inconvenient to move in medical locations.

Further, mobile computational devices are commonly known and have spread throughout clinical applications in the last couple of years. Nowadays, mobile phones, tablets, personal computers and notebooks are largely used to provide all kinds of applications and network access independent of their location. These mobile devices have steadily increasing hardware performance levels, easy to use interfaces, and displays with increasing resolution and quality.

For example, reference JP 2007129342 A discloses a mobile phone with a personal computer function including an interface connectable to an external device for storing personal computer operating systems, an internal storage means for storing a mobile phone operating system, a controlling means for controlling execution of a program on the basis of the mobile phone operating system, a judgment means for judging whether or not the external device is connected to the interface, and a switching means for switching the mobile phone operating system to the personal computer operating system when the judgment means judges that the external device is connected to the interface, wherein the control means controls execution of the program on the basis of the personal computer operating system when the personal computer operating system is selected.

US 2004/0179332 A1 discloses a portable ultrasound unit and a docking cart for the unit, wherein a portable ultrasound unit is mounted to the docking cart and the docking cart transforms the portable ultrasound unit into a cart-based system with enhanced features and functionality. A clinician display and patient display may be provided on the cart.

US 2003/0139664 A1, which forms the basis for the preamble of claim 1, discloses a segmented ultrasound system, in which ultrasound data, such as image data in a video format, is wirelessly transmitted to a multi-use display device from a handheld ultrasound device.

There is a need to further improve ultrasound imaging systems in terms of costs, portability and multipurpose functionality.

### SUMMARY OF THE INVENTION

It is an object of the present invention to provide an improved ultrasound image acquisition station and an improved ultrasound imaging system.

In a first aspect of the present invention, an ultrasound imaging system for providing an image of a tomographic plane or volume is presented, comprising an ultrasound image acquisition station and a mobile display device, wherein the ultrasound image acquisition station comprises a transducer array configured to provide an ultrasound receive signal, an image acquisition hardware assembly for controlling the transducer array, receiving the ultrasound receive signal and providing image data, a first memory unit having stored thereon a first operating system for operating the ultrasound imaging system, and an interface assembly for removable connecting the mobile display device with the ultrasound image acquisition station, wherein the mobile display device has a central processing unit and a second memory unit having stored thereon a second operating system for operating the mobile display device, wherein the mobile display device is connectable to the interface assembly, and wherein the ultrasound imaging system is configured such that, upon connection of the mobile display device to the interface assembly, the second operating system is bypassed and the mobile display device is operated by the first operating system, wherein the first operating system takes over control of the central processing unit of the mobile display device.

In a further aspect of the present invention an ultrasound imaging system for providing an image of area tomographic or volume is presented that comprises an ultrasound image acquisition station comprising a transducer array configured to provide an ultrasound receive signal, an image acquisition hardware assembly for controlling the transducer array, receiving the ultrasound receive signal and providing image data, a first memory unit having stored thereon a first operating system for operating the ultrasound imaging system, and an interface assembly for connecting the mobile display device with the ultrasound image acquisition station, and wherein the ultrasound imaging system further comprises a mobile display device, wherein the mobile display device has a second memory unit having stored thereon a second operating system for operating the mobile display device, and wherein the mobile display device is connectable to the interface assembly, wherein the ultrasound imaging system is configured such that the second operating system is bypassed and the mobile display device is operated by the first operating system upon connection of the mobile display device to the interface assembly.

The basic idea of the invention is to make use of the hardware resources and the portability of widespread mobile display devices to enable commanding of ultrasound imaging systems and processing ultrasound image data.

It is contemplated to temporarily replace an operating system of a mobile display device to provide a dedicated ultrasound imaging function for a defined period. The commandeered central processing unit and rendering hardware of the mobile display device run the ultrasound application exclusively, yielding higher performance and diagnostic reliability. When scanning is initiated, the ultrasound operating system is inserted into the mobile display device. When scanning is completed, the mobile display device is returned to its original operating system and function.

By this, a single target mobile display device can safely serve multiple purposes, one of which is restricted to exclusive ultrasound image processing. The other purpose may be for the common general purposes of the user. Further, these general purposes may include additional medical purposes, for example within a clinic or other location for medical aid. Having a single mobile display device reduces the costs of ownership and the clutter of otherwise dedicated scanning devices. Further, the effective portability of the ultrasound imaging system is enhanced, since the user is already carrying his/her personal computer. Because the ultrasound imaging application enjoys sole access to the resources of the mobile display device, it can reliably claim to generate valid evaluable ultrasound images and to store or transfer those images securely.

In particular, the first memory unit is a non-transitory storage device on which data executing a first operating system for operating all hardware components of the ultrasound imaging system is stored. By this, the first memory unit presents an alternate boot drive to the mobile display device. Preferably, there is a rapid start-up provided by a mechanism such as the Hibernate-Once-Resume-Many (HORM) boot sequence. Such a HORM feature allows for a rapid reboot and return to the same hibernated operation over and over again. A version of an ultrasound operating system may be rapidly booted from the predefined hibernation state. The ultrasound imaging system can be booted to an operating state from zero-power-standby in approximately seven seconds.

Hence, there is provided the ultrasound image acquisition station comprising all hardware necessary for an ultrasound image acquisition. Further, it is configured to connect to a mobile display device a first memory unit having stored thereon the first operating system. In particular, the first memory unit may be a flash memory device. Further, the ultrasound image acquisition station may comprise a judging means for judging if a mobile display device is connected to the ultrasound image acquisition station via the interface assembly.

Furthermore, the ultrasound image acquisition station may comprise switching means for switching, in particular automatically switching, the operation of the mobile display device towards operation via the first operating system upon judging that a mobile display device is connected to the ultrasound image acquisition station by the judging unit. The ultrasound image acquisition station may be in the form of a standard attachment to a mobile display device via its docking port. It may be a kind of a sleeve surrounding the mobile display device and carried with the mobile display device, or may have the form of a typical table-top docking station, or may have the form of a docking port connector cabled to or part of a smart probe carrying the transducer array, the image acquisition hardware assembly and the first memory unit. Hence, in the case of a smart probe, all ultrasound specific hardware, i.e. at least the beam former, and, optionally, the signal processor and, further optionally the image processor, may be contained in the handle of the probe, including the first memory unit containing the first operating system. The smart probe or the image acquisition station may connect to the mobile display device by means of a custom wired interface, a standard wired interface such as universal serial bus (USB), USB 2.0, USB 3.0 or a wireless interface such as wireless USB on an ultra-wideband (UWB) radio link. In the case of a wireless smart probe, a battery may also be included in the smart probe, and the entire smart probe should optimally consume less than an average of 3.5 watts during scanning operation.

Preferred embodiments of the invention are defined in the dependent claims.

In an embodiment, the image acquisition hardware assembly has a beam former configured to control the transducer array to scan area two-dimensional tomographic plane or a three-dimensional volume, in particular of a body of a patient, and further configured to receive the ultrasound receive signal and to provide an image signal, and a signal processor configured to receive the image signal and to provide image data. By this, the ultrasound image acquisition station can provide full ultrasound hardware functionality to command the transducer array and can provide image data to the mobile display device via the interface assembly that can be further processed by the mobile display device. All ultrasound specific hardware and/or software components are provided in the ultrasound image acquisition station.

In a further embodiment, the ultrasound image acquisition station further comprises a first input device for enabling a user to command the ultrasound imaging system. By this, further possibilities to input data and to the ultrasound imaging system and to control the ultrasound imaging system can be provided to a user. For example, in case the mobile display device only has a touch screen, a keyboard can be provided to the user via the input device of the ultrasound image acquisition system to enable the user to readily input the name of the patient, information related to the ultrasound images, etc. via the keyboard. Further, the first input device may comprise a trackball for steering a mouse pointer on the mobile display device, or to scan, enlarge and rotate a rendered three-dimensional image in case a volume is scanned via the ultrasound imaging system. For example, physical buttons implementing basic ultrasound functions such as to start and stop active imaging, or to set the image depth, may be included in the ultrasound image acquisition station. In general, all input possibilities can be provided to a user further to the input possibilities that may be provided by a mobile display device.

In a further embodiment, the ultrasound image acquisition station further comprises a docking unit comprising the image acquisition hardware assembly, the first memory unit and the interface assembly. By this, the ultrasound image acquisition station can be provided to the user as a docking unit for receiving the mobile display device. Placing the mobile display device in the bottom station can provide an intuitive and apparently easy procedure for connecting the mobile display device with the ultrasound image acquisition station.

In a further embodiment, the ultrasound image acquisition station further comprises a probe head that comprises the transducer array. By this, usual probe heads can be connected to the docking unit. Further, the patient may be scanned manually in case the probe head can be manually operated. However, it may also be the case that the probe head is part of a more complex device that may be placed on a portion of a body of a patient to automatically scan a larger volume of the patient, for example the torso, the heart or a breast of a patient.

In a further embodiment, the probe head is cable-connected to the docking station. By this, a reliable connection being able to provide for the transmittal of large bandwidth can be provided.

In a further embodiment, the ultrasound image acquisition station further comprises an adapter unit, wherein the interface assembly is configured as a plug and socket connection, and wherein the adapter unit is designed to enable connection of a specific type of mobile display device to the interface assembly. By this, the mobile display device may be easily connected to the ultrasound image acquisition station and its docking unit. The adapter unit may facilitate connection of the mobile display device. Further, the adapting unit may serve to securely hold the mobile display device attached to the docking unit during operation.

In a further embodiment, the docking unit is generally L-shaped in cross section. By this, the docking unit may be provided in the form of a plate with a smooth angled foot feature in the back which forms a combination of hook, stand and transducer interface. One or more, preferably two, interfaces for transducer arrays may be provided within the angled feature of the docking unit.

By by passing the second operating system, the mobile display device becomes dedicated to the ultrasound scanner function when docked. The mobile display device will be booted into the hibernated state of the first operating system within seconds, providing the full hardware resources to the ultrasound imaging procedure.

In a further embodiment, the mobile display device comprises a central processing unit for controlling the beam former, an image processor configured to receive the image data from the signal processor and to provide display data, the display configured to receive the display data and to provide the image. By this, the mobile display device comprises the necessary hardware resources to command the ultrasound image acquisition station. Further, the received image data can be processed by a central processing unit and resources of the mobile display device and rendered to display display data on the display of the mobile display device.

In a further embodiment, the mobile display device further comprises a second input device. By this, a user may still turn to the input device of the mobile display device in order to command the ultrasound imaging system. A second input device may for example be a touch screen of the mobile display device, or buttons provided on the mobile display device.

In a further embodiment, the ultrasound imaging system is configured such that the mobile display device is operated by the second operating system when the mobile display device is disconnected from the ultrasound image acquisition station. By this, a user may readily turn the mobile display device back into the general purpose state by disconnecting the mobile display device from the interface assembly of the ultrasound image acquisition station. Further to such automatic switching procedure, the user may be provided with an option to switch between the first and second operating systems during connection of the mobile display device to the ultrasound image acquisition station. For example, such an option may be provided by touching a certain button of the first or second input device or selecting a certain program in the respective operating system architecture.

In a further embodiment, the mobile display device further comprises a signature unit to identify the mobile display device to the ultrasound image acquisition station and to enable bypassing the second operating system and operating the mobile display device by the first operating system. By this, only valid mobile display devices may be connected to the ultrasound acquisition station. This may ensure that only such mobile display devices are connected to the ultrasound image acquisition station that have sufficient hardware resources to run the first operating system. Further, this may ensure that only certain devices of a specific manufacturer, a specific clinic or selected personal may be connected to a certain ultrasound image acquisition station.

In a further embodiment, the mobile display device is a tablet type personal computer or a clamshell type personal computer. By this, commonly known types of personal computers and mobile display devices may be connected to the ultrasound image acquisition station to form the ultrasound imaging system.

In a further embodiment, the ultrasound imaging system is further configured such that at least a portion of the first operating system is copied to the second memory unit of the mobile display device, in particular under control of the first operating system of the image acquisition assembly. By this, the interface link between the image acquisition assembly and the mobile display device may then have a lower transfer rate than would normally be required for the frequent access by the first operating system to its source memory in the first memory unit. Hence, more bandwidth is available, for example for the transfer of ultrasound image data or ultrasound display data.

### BRIEF DESCRIPTION OF THE DRAWINGS

These and other aspects of the invention will be apparent from and elucidated with reference to the embodiment(s) described hereinafter. In the following drawings
Fig. 1 shows a schematic illustration of an application of an ultrasound imaging system,
Fig. 2 shows a schematic block diagram of an ultrasound imaging system and of an ultrasound image acquisition system connectable to a mobile display device,
Fig. 3 shows an ultrasound imaging system with an ultrasound image acquisition station connected to a mobile display device of a tablet type personal computer, and
Fig. 4 shows an ultrasound imaging system comprising an ultrasound image acquisition station connected to a clamshell type personal computer.

### DETAILED DESCRIPTION OF THE INVENTION

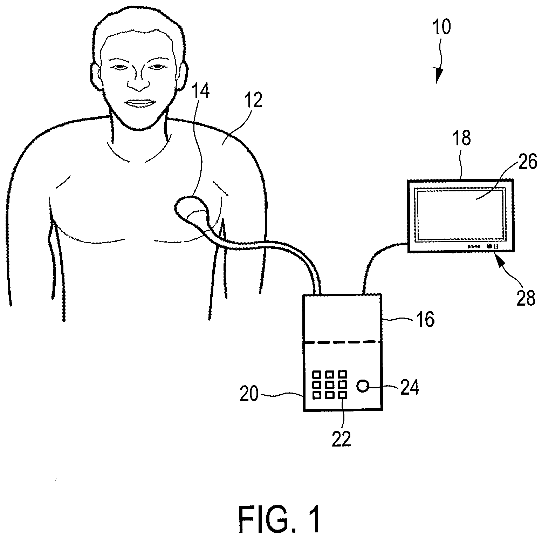

Fig. 1 shows an ultrasound imaging system 10. The ultrasound imaging system 10 is used for scanning an area or volume of the body of the patient 12.

For scanning the patient 12, a probe head 14 may be provided. The probe head 14 is connected to a docking unit 16 of the ultrasound imaging system 10. The docking unit 16 serves not only for connecting the probe head 14 but also for connecting a mobile display device 18. While the mobile display device 18 is connected to the docking unit 16 via a cable shown in Fig. 1, it is contemplated that the mobile display device 18 is connected to the docking unit 16 via a plug and socket connection, or may be easily connected in a wireless manner to the docking unit 16.

The docking unit 16 may comprise a first input device 20. The first input device 20 may have a keypad 22 and/or a trackball 24 to provide an input mechanism to a user of the ultrasound imaging system 10. Additionally or alternatively, other mechanisms may be present in the first input device 20 to enable a user to control the ultrasound imaging system 10.

Further, the mobile display device 18 comprises a display unit 26 to display display data generated by the ultrasound imaging system to the user. By this, the area or volume of the patient 12 that is scanned via the probe head 14 can be viewed on the display unit 26 by the user of the ultrasound imaging system 10. Further, the mobile display device 18 comprises a second input device 28 that may provide additional control possibilities to the user. The second input device may for example be the display unit being provided as a touch screen or additional buttons present on the mobile display device 18.

The "mobile display device" 18 may be any computational hardware device that may be carried by a user. In particular, the mobile display device 18 may be a cell phone, a PDA (Personal Digital Assistant), a clamshell type personal computer or a tablet type personal computer.

The mobile display device 18 may be connected and disconnected to the docking unit 16 by the user of the ultrasound imaging system 10. When disconnected from the docking unit 16, the mobile display device 18 may be run with its own operating system providing all kinds of general purpose applications or medical applications to the user. Further, upon docking the mobile display device 18 to the docking unit 16, the operating system of the mobile display device 18 is bypassed and an operating system of the ultrasound imaging system 10 stored in the docking unit 16 is automatically booted from a hibernated state. This alternative operating system immediately takes over control of the mobile display device 18 and provides the user with a fully functioning ultrasound imaging system 10 using the hardware resources of the mobile display device 18.

In a further embodiment, it is also possible that the probe head 14 and the docking unit 16 form a single entity, i.e. are contained within the same housing. Hence, the probe head 14 and the docking unit may form a single package in a probe housing.

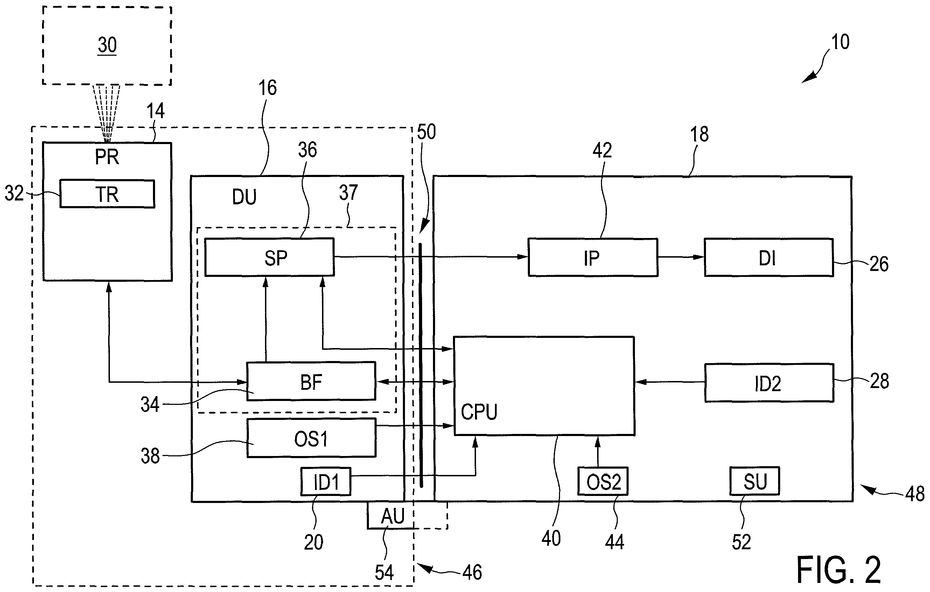

Fig. 2 shows a schematic block diagram as an example for the various components of the ultrasound imaging system 10 and their location and interaction within the whole ultrasound imaging system 10.

As already explained above, the ultrasound imaging system 10 is used for scanning an area or volume 30 of a patient 12. The area or volume is schematically shown in dashed lines and designated with reference numeral 30. The area or volume 30 is examined via the probe head 14 carrying a transducer array 32. The transducer array 32 may be of any known type. Hence, the transducer array 32 may be a one-dimensional transducer array or a two-dimensional transducer array that may be mechanically or electronically scanned. The transducer array 32 converts the ultrasound signals into electronic signals and vice versa.

To command the transducer array 32, a beam former 34 is present that is used to control the electronic and/or mechanical scanning of the transducer array and, if possible, the number, density and position of scan lines along which the area or volume 30 is scanned. Further, a signal processor may be provided that receives the ultrasound receive signal of the beam former and provides an image signal. The beam former 34 and the signal processor 36 together may form an image acquisition hardware 37. Further, there is provided a first memory unit 38 having stored thereon a first operating system that operates all hardware of the ultrasound imaging system 10 in ultrasound imaging applications only.

The mobile display device 18 comprises a central processing unit 40 for controlling the beam former 34, an image processor 42 and/or the signal processor 36. In any embodiment, the beam former 34, the signal processor 36 and the image processor 42 may also be of a software-implemented type being part of the first operating system 38 and run on the central processing unit 40. In case one of the group of the signal processor 36, the beam former 34 and the image processor 42 is of a hardware-implemented type, the location of the respective circuitry is preferably as shown in Fig. 2. However, it might also be the case that hardware circuitry shown as part of the docking unit 16 may be present on the mobile display device 18, or hardware components as present on the mobile display device 18 may be part of the docking unit 16.

The image processor 42 receives image data from the signal processor 36 and provides display data to the display 26. The beam former 34, the signal processor 36 and the image processor 42 may be run by the central processing unit 40.

Further, the mobile display device 18 carries a second memory unit 44 having stored thereon a second operating system to run the mobile display device in a general purpose state. By this, if disconnected from the docking unit 16, the mobile display device 18 can be used by a user for any application. Connected to docking unit 16, the first operating system stored on the first memory unit 38 takes over control of the central processing unit 40 and/or hardware resources of the ultrasound imaging system 10.

The first memory unit 38 and the second memory unit 44 may comprise at least one hard disk, for example a solid state drive (SSD), and may further comprise at least one random access memory (RAM) device.

In any case, the probe head 14 and the docking unit 16 together form an ultrasound image acquisition station 46. This ultrasound image acquisition station 46 is capable of being connected to a console portion 48 of the ultrasound imaging system 10 in which console portion 48 is formed by the mobile display device 18. In a further embodiment, the ultrasound image acquisition station 46 may be formed in a single housing. Hence, the transducer array 32, the beam former 34 and the signal processor 36 may all be located in the same housing as the probe head 14. Further, the first memory unit 38 can also be located within that housing.

To enable the connection of the mobile display device 18 to the ultrasound image acquisition station 46, there is provided an interface assembly 50, preferably in the form of a plug and socket connection. An interface assembly 50 forms part of the ultrasound image acquisition station 46 to receive a mobile display device 18 for docking. To ensure that only proper mobile display devices 18 are connected to the ultrasound image acquisition station 46, the mobile display device 18 may comprise a signature unit 52 for identifying a valid mobile display device 18 to the ultrasound image acquisition station 46.

To properly connect a mobile display device 18 to the docking unit 16, an adapter unit 54 may be provided that may serve as a frame and/or guidance to insert and hold the mobile display device 18 within docking unit 16. Further, via the adapter unit 54, a flush arrangement of the mobile display device 18 within the docking unit 16 may be provided to facilitate operation of the ultrasound imaging system 10.

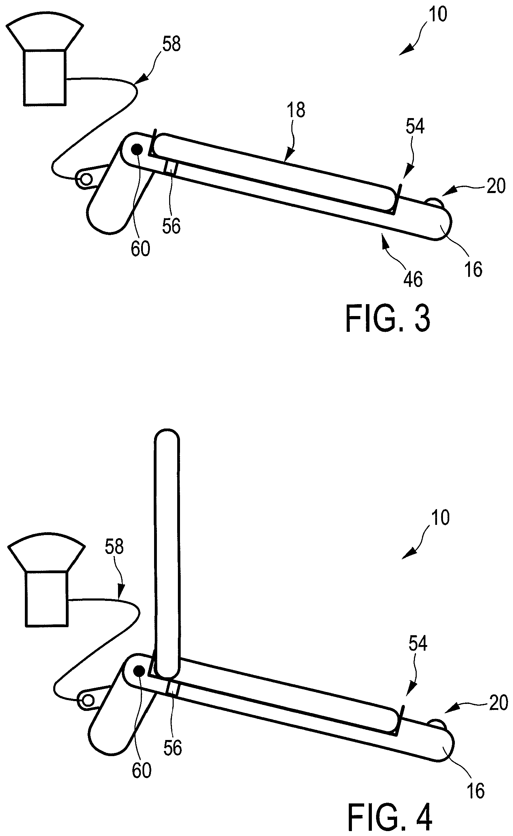

Fig. 3 shows a sketch of a possible practical embodiment of the ultrasound imaging system 10 and its components. The docking unit 16 has a schematically depicted plug and socket connection 56 to provide the data connection to the mobile display device 18. In the embodiment shown in Fig. 3, the mobile display device is a tablet type personal computer. The probe head 14 is attached via cable 58 to the docking unit 16. However, even two or more probe heads 14 may be attached to the docking unit 16. The docking unit 16 has an L-shaped cross section with a relatively long feature comprising the docking unit 54 and the first input device 20 and a short feature which comprises the interface for connecting the probe head 14. Both features are connected via a hinge, so that the docking unit 16 may be put into a convenient position.

The docking unit 16 is a compact portable low cost secure and versatile module that may convert any mobile tablet PC into a feature rich ultrasound scanner in particular aimed at the lower end market segment. It consists of an ergonomic frame and the mobile display device 18 may snap-lock into the plug and socket connection 56. The docking unit may contain an optionally battery part image acquisition hardware 37. The operating system stored on the mobile display device may for example be a Windows operating system, an Android operating system or an iPhone iOS operating system.

The probe head 14 may comprise a transducer array 32 of any known type, for example it may be a linear, curved linear array (CLA), sector or endo-cavity type transducer array. The ultrasound image acquisition station 46 may contain an auxiliary battery that may supplement a battery present in the mobile display device. By this, the ultrasound imaging system may be allowed for scanning over a time period of several hours. Further, a power receptable may be present that may provide a DC input from an AC wall adapter to allow the system to scan without time limit.

Further, the docking unit may provide a video output and port expansion such as WiFi and LAN. It may contain a 100 and more Gigabyte solid state drive (SSD) hard disk as the first memory unit having stored the operating system of the ultrasound imaging system 10 stored thereon. Further, the first memory unit 38 may provide image data storage capacity. The mobile display device itself may provide for example WiFi and Bluetooth connections. The docking unit 16 may be designed to be portable, weighing less than 2 kg. While scanning a body of a patient, the docking unit 16 may be laid on a relatively flat surface or mounted on a stand or on the wall.

The mobile display device may for example be run by Dual Core Atom Processor commercially available from the Intel company, Santa Clara, USA. However, any other central processing unit may be present. If not connected to the docking unit, the mobile display device may be an open system to suit any users' needs, for example web browsing, e-mail, other medical applications, video, personal instant messaging, work processing, etc. A user may be free to install additional software and upgrade the mobile display device. In particular, the mobile display device may meet the typical specifications of the standard Medical Clinical Assistant architecture. Such an architecture may be present to provide tablet PC infrastructure for the use of medical personnel at clinics.

When the mobile display device 18 is docked to the docking unit 16, however, the ultrasound image acquisition station 46 takes over all of the mobile display devices, hardware resources and bypasses the second operating system 44 within the mobile display device. This is accomplished by booting again from the first memory unit 38 in the ultrasound image acquisition station 46. An option may be provided to copy images stored on the first memory unit 38 to the mobile display device's second memory unit 44 for review when the mobile display device 18 is disconnected from the docking unit 16. Without the mobile display device, the docking unit 16 may not boot from its internal memory unit 38 and will not power the probe head 14. Such a mechanism may ensure that only skilled personnel may operate the ultrasound imaging system 10.

Further, the ultrasound imaging system 10 may be configured such that upon connection of the mobile display device 18 to the docking unit 16 the first operating system is booted from the first memory unit 38 in the docking unit 16, the second operating system in the mobile display device 18 is bypassed, and at least a portion of the first operating system is copied to a volatile memory, for example a RAM device of the second memory unit 44, of the mobile display device 18 under control of the first operating system of the image acquisition assembly 46. By this, an ultrasound imaging program can be executed more efficiently, without requiring frequent access to the first memory unit 38 of the image acquisition assembly 46 over the interface assembly 50 between the image acquisition assembly 46 and the mobile display device 18. Copying a portion of the first operating system of the ultrasound imaging system 10 to a volatile memory or RAM device of the second memory unit 44 of the mobile display device 18 has no effect on the un-docked operation of the mobile display device 18. The advantage of doing so is that the interface link between the image acquisition assembly 46 and the mobile display device 18 may then have a lower transfer rate, e.g. in Mbits/sec, than would normally be required for the frequent access by the first operating system to its source memory in the first memory unit 38. This may be particularly useful if an USB 2.0 standard is used for the interface assembly 50.

However, if for example USB 3.0 is used as a standard for the interface assembly 50, this would offers about ten times the transfer rate of USB 2.0,. Hence using USB 3.0 could obviate the copying of at least a portion of the first operating system into the second memory unit 44, in particular the RAM, of the mobile display device 18, and allow the mobile display device 18 to access the first memory unit 32 in the ultrasound image acquisition station 46 continuously.

As commercially available mobile display devices 18 change over the time, the adapter unit 54 is provided at the docking unit 16. The adapter unit 54 may be exchanged within the docking unit. The adapter unit 54 is designed to conform to the outside plastics of the mobile display device. The adapter may not be removable by the user but only by skilled personnel. The adapter unit may also contain the corresponding plug and socket connection 56. Such a connection may join power, USB, PCIe, LAN and video signals to the docking unit, so all components of the mobile display device 18 and the ultrasound image acquisition station 46 may be exchanged via the interface assembly 50 provided by the plug and socket connection. Further external connectors may be provided on the docking unit 16, for example a power jack, LAN, video and USB.

In general, the mobile display device 18 may remain locked to the docking unit 16 over a larger time period to provide for a stationary ultrasound imaging system 10.

As shown in Fig. 4, the mobile display device 18 must not necessarily be a tablet personal computer but could alternatively be in the form of a clamshell type personal computer. It may also be a kind of a cell phone, smart phone or personal digital assistant.

In general, the contemplated embodiments provide for a compact design with small dimensions and a low weight. The ultrasound imaging system 10 is portable and may be hand-carried but also laid on a surface while operating. It may also be permanently mounted to a rolling stand. The mobile display device 18 may serve as a user's own personal device or, when connected to the ultrasound image acquisition station 46, as a dedicated ultrasound scanner. The operating system may be set in a way that the ultrasound software application and data are entirely separate from the user's software and data. The mobile display device 18 may be locked to the docking unit 16 for physical security. Alternatively, the mobile display device 18 can be removed and secured separately to protect its replacement value or its data. Via the signature unit 52, the ultrasound image acquisition station 46 may only operate when connected to a valid mobile display device 18. Further, the ultrasound imaging system 10 maybe provided at low cost since the mobile display device 18 may be one of commercially available mobile display devices produced in high numbers.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. An ultrasound imaging system (10) for providing an image of a tomographic plane or volume (30), comprising an ultrasound image acquisition station (46) and a mobile display device (18), the ultrasound image acquisition station (46) comprising:
(i) a transducer array (32) configured to provide an ultrasound receive signal,
(ii) an image acquisition hardware assembly (37) for controlling the transducer array (32), receiving the ultrasound receive signal and providing image data,
(iii) a first memory unit (38) having stored thereon a first operating system for operating the ultrasound imaging system (10), and
(iv) an interface assembly (50) for removable connecting the mobile display device (18) with the ultrasound image acquisition station (46),
wherein the mobile display device (18) has a central processing unit (40) and a second memory unit (44) having stored thereon a second operating system for operating the mobile display device (18), and wherein the mobile display device (18) is connectable to the interface assembly (50),
**characterized in that** the ultrasound imaging system (10) is configured such that, upon connection of the mobile display device (18) to the interface assembly (50), the second operating system is bypassed and the mobile display device (18) is operated by the first operating system, wherein the first operating system takes over control of the central processing unit (40) of the mobile display device (18).

2. The ultrasound imaging system (10) according to claim 1, wherein the image acquisition hardware assembly (37) has a beam former (34) configured to control the transducer array (32) to scan a tomographic plane or volume (30), and further configured to receive the ultrasound receive signal and to provide an image signal, and a signal processor (36) configured to receive the image signal and to provide image data.

3. The ultrasound imaging system (10) according to claim 1, wherein the ultrasound image acquisition station (46) further comprises a first input device (20) for enabling a user to command the ultrasound imaging system (10).

4. The ultrasound imaging system (10) according to claim 1, wherein the ultrasound image acquisition station (46) further comprises a docking unit (16) comprising the image acquisition hardware assembly (37), the first memory unit (38) and the interface assembly (50).

5. The ultrasound imaging system (10) according to claim 1, wherein the ultrasound image acquisition station (46) further comprises a probe head (14) that comprises the transducer array (32).

6. The ultrasound imaging system (10) according to claim 5, wherein the probe head (14) is cable-connected to the interface assembly (50).

7. The ultrasound imaging system (10) according to claim 1, wherein the ultrasound image acquisition station (46) further comprises an adapter unit (52), wherein the interface assembly (50) is configured as a plug and socket connection, and wherein the adapter unit (52) is designed to enable connection of a specific type of mobile display device (18) to the interface assembly (50).

8. The ultrasound imaging system (10) according to claim 4, wherein the docking unit (16) is generally L-shaped in cross-section.

9. The ultrasound imaging system (10) according to claim 1, wherein the mobile display device (18) further comprises a second input device (28).

10. The ultrasound imaging system (10) according to claim 1, wherein the ultrasound imaging system (10) is configured such that the mobile display device (18) is operated by the second operating system when the mobile display device (18) is disconnected from the interface assembly (50).

11. The ultrasound imaging system (10) according to claim 1, wherein the mobile display device (18) further comprises a signature unit (52) to identify the mobile display device (18) to the ultrasound image acquisition station (46) and to enable bypassing the second operating system and operating the mobile display device (18) by the first operating system.

12. The ultrasound imaging system (10) according to claim 1, wherein the ultrasound imaging system (10) is further configured such that at least a portion of the first operating system is copied to the second memory unit (44) of the mobile display device (18) under control of the first operating system of the image acquisition assembly (46).

## Patentansprüche

1. Ein Ultraschallbildgebungssystem (10) zum Bereitstellen eines Bilds von tomografischen Ebenen oder Volumen (30), das eine Ultraschall-Bilderfassungsstation (46) sowie ein mobiles Anzeigegerät umfasst (18), wobei die Ultraschall-Bilderfassungsstation (46) Folgendes umfasst:
(i) eine Wandleranordnung (32), die ein Ultraschallempfangssignal bereitstellt,
(ii) eine Bilderfassungs-Hardwarebaugruppe (37) zum Steuern der Wandleranordnung (32), die das Ultraschallempfangssignal abruft und Bilddaten bereitstellt,
(iii) eine erste Speichereinheit (38), auf der ein erstes Betriebssystem für das Ausführen des Ultraschallbildgebungssystem (10) gespeichert ist, und
(iv) eine Schnittstellenbaugruppe (50) zum trennbaren Anschließen des mobilen Anzeigegeräts (18) an der Ultraschall-Bilderfassungsstation (46), wobei das mobile Anzeigegerät (18) über eine zentrale Verarbeitungseinheit (40) und eine zweite Speichereinheit (44) verfügt, auf der ein zweites Betriebssystem zum Ausführen des mobilen Anzeigegeräts (18) gespeichert ist, und wobei das mobile Anzeigegerät (18) an der Schnittstellenbaugruppe (50) angeschlossen werden kann,
das sich dadurch auszeichnet, dass Ultraschallbildgebungssystem (10) beim Anschließen des mobilen Anzeigegeräts (18) an der Schnittstellenbaugruppe (50) das zweite Betriebssystem umgeht, sodass das mobile Anzeigegerät (18) vom ersten Betriebssystem ausgeführt wird, wobei das erste Betriebssystem die Steuerung der zentralen Verarbeitungseinheit (40) des mobilen Anzeigegeräts (18) übernimmt.

2. Das Ultraschallbildgebungssystem (10) gemäß Anspruch 1,
wobei die Bilderfassungs-Hardwarebaugruppe (37) über einen Beamformer (34), der die Wandleranordnung (32) steuert, um eine tomografische Ebene oder ein Volumen (30) zu scannen, und der zudem das Ultraschallempfangssignal abruft und ein Bildsignal bereitstellt, sowie über einen Signalprozessor (36) verfügt, der das Bildsignal abruft und Bilddaten bereitstellt.

3. Das Ultraschallbildgebungssystem (10) gemäß Anspruch 1,
wobei die Ultraschall-Bilderfassungsstation (46) zudem über ein erstes Eingabegerät (20) verfügt, über das der Benutzer Befehle für das Ultraschallbildgebungssystem (10) eingeben kann.

4. Das Ultraschallbildgebungssystem (10) gemäß Anspruch 1,
wobei die Ultraschall-Bilderfassungsstation (46) zudem über eine Docking-Einheit (16) mit der Bilderfassungs-Hardwarebaugruppe (37), der ersten Speichereinheit (38) und der Schnittstellenbaugruppe (50) verfügt.

5. Das Ultraschallbildgebungssystem (10) gemäß Anspruch 1,
wobei die Ultraschall-Bilderfassungsstation (46) zudem über einen Sondenkopf (14) mit der Wandleranordnung (32) verfügt.

6. Das Ultraschallbildgebungssystem (10) gemäß Anspruch 5,
wobei der Sondenkopf (14) über ein Kabel an der Schnittstellenbaugruppe (50) angeschlossen ist.

7. Das Ultraschallbildgebungssystem (10) gemäß Anspruch 1,
wobei die Ultraschall-Bilderfassungsstation (46) zudem über eine Adaptereinheit (52) verfügt, und wobei die Schnittstellenbaugruppe (50) über einen Stecker-Buchsen-Anschluss verfügt, und wobei die Adaptereinheit (52) das Anschließen eines bestimmten Typs von mobilem Anzeigegerät (18) an der Schnittstellenbaugruppe (50) ermöglicht.

8. Das Ultraschallbildgebungssystem (10) gemäß Anspruch 4,
wobei die Docking-Einheit (16) in der Regel über einen L-förmigen Querschnitt verfügt.

9. Das Ultraschallbildgebungssystem (10) gemäß Anspruch 1,
wobei das mobile Anzeigegerät (18) zudem über ein zweites Eingabegerät verfügt (28).

10. Das Ultraschallbildgebungssystem (10) gemäß Anspruch 1,
wobei das Ultraschallbildgebungssystem (10) so konfiguriert ist, dass das mobile Anzeigegerät (18) über das zweite Betriebssystem betrieben wird, wenn das mobile Anzeigegerät (18) von der Schnittstellenbaugruppe (50) getrennt ist.

11. Das Ultraschallbildgebungssystem (10) gemäß Anspruch 1,
wobei das mobile Anzeigegerät (18) zudem über eine Signatureinheit (52) verfügt, um das mobile Anzeigegerät (18) für die Ultraschall-Bilderfassungsstation (46) zu identifizieren und das Umgehen des zweiten Betriebssystems zu ermöglichen, sodass das mobile Anzeigegerät (18) vom ersten Betriebssystem ausgeführt wird.

12. Das Ultraschallbildgebungssystem (10) gemäß Anspruch 1,
wobei das Ultraschallbildgebungssystem (10) zudem so konfiguriert ist, dass mindestens ein Teil des ersten Betriebssystems auf die zweite Speichereinheit (44) des mobilen Anzeigegeräts (18) kopiert wird, die vom ersten Betriebssystem der Bilderfassungsbaugruppe (46) ausgeführt wird.

## Revendications

1. Système d'imagerie par ultrasons (10) permettant de fournir une image d'un plan ou d'un volume tomographique (30), comprenant une station d'acquisition d'images par ultrasons (46) et un dispositif d'affichage (18) mobile, ladite station d'acquisition d'images par ultrasons (46) comprenant :
(i) un réseau de transducteurs (32) configuré pour fournir un signal de réception ultrasonore,
(ii) un ensemble matériel d'acquisition d'images (37) pour commander le réseau de transducteurs (32), pour recevoir le signal de réception ultrasonore et pour fournir des données d'image,
(iii) une première unité de mémoire (38) dans laquelle est mémorisé un premier système d'exploitation pour faire fonctionner le système d'imagerie par ultrasons (10), et
(iv) un ensemble interface (50) pour connecter de manière amovible le dispositif d'affichage (18) mobile à la station d'acquisition d'images par ultrasons (46),
dans lequel le dispositif d'affichage (18) mobile comporte une unité centrale de traitement (40) et une seconde unité de mémoire (44) dans laquelle est mémorisé un second système d'exploitation pour faire fonctionner le dispositif d'affichage (18) mobile, et dans lequel le dispositif d'affichage (18) mobile peut être connecté à l'ensemble interface (50), **caractérisé en ce que** le système d'imagerie par ultrasons (10) est configuré de telle sorte que, lors de la connexion du dispositif d'affichage (18) mobile à l'ensemble interface (50), le second système d'exploitation est contourné et le dispositif d'affichage (18) mobile est actionné par le premier système d'exploitation, dans lequel le premier système d'exploitation prend la commande de l'unité centrale de traitement (40) du dispositif d'affichage (18) mobile.

2. Système d'imagerie par ultrasons (10) selon la revendication 1, dans lequel l'ensemble matériel d'acquisition d'images (37) comporte un formateur de faisceau (34) configuré pour commander le réseau de transducteurs (32) pour balayer un plan ou un volume tomographique (30), et en outre configuré pour recevoir le signal de réception ultrasonore et pour fournir un signal d'image et un processeur de signal (36) configuré pour recevoir le signal d'image et pour fournir des données d'image.

3. Système d'imagerie par ultrasons (10) selon la revendication 1, dans lequel la station d'acquisition d'images par ultrasons (46) comprend en outre un premier dispositif d'entrée (20) pour permettre à un utilisateur de commander ledit système d'imagerie par ultrasons (10).

4. Système d'imagerie par ultrasons (10) selon la revendication 1, dans lequel la station d'acquisition d'images par ultrasons (46) comprend en outre une unité d'amarrage (16) comprenant l'ensemble matériel d'acquisition d'images (37), la première unité de mémoire (38) et l'ensemble interface (50).

5. Système d'imagerie par ultrasons (10) selon la revendication 1, dans lequel la station d'acquisition d'images par ultrasons (46) comprend en outre une tête de sonde (14), laquelle comprend le réseau de transducteurs (32).

6. Système d'imagerie par ultrasons (10) selon la revendication 5, dans lequel la tête de sonde (14) est connectée par câble à l'ensemble interface (50).

7. Système d'imagerie par ultrasons (10) selon la revendication 1, dans lequel la station d'acquisition d'images par ultrasons (46) comprend en outre une unité d'adaptateur (52), dans lequel l'ensemble interface (50) est conçu sous la forme d'une connexion à fiche, et dans lequel l'unité d'adaptateur (52) est conçue pour permettre la connexion d'un type spécifique de dispositif d'affichage (18) mobile à l'ensemble interface (50).

8. Système d'imagerie par ultrasons (10) selon la revendication 4, dans lequel l'unité d'amarrage (16) est généralement en forme de L en coupe transversale.

9. Système d'imagerie par ultrasons (10) selon la revendication 1, dans lequel le dispositif d'affichage (18) mobile comprend en outre un second dispositif d'entrée (28).

10. Système d'imagerie par ultrasons (10) selon la revendication 1, dans lequel le système d'imagerie par ultrasons (10) est configuré de telle sorte que le dispositif d'affichage (18) mobile est actionné par le second système d'exploitation lorsque le dispositif d'affichage (18) mobile est déconnecté de l'ensemble interface (50).

11. Système d'imagerie par ultrasons (10) selon la revendication 1, dans lequel le dispositif d'affichage (18) mobile comprend en outre une unité de signature (52) pour identifier le dispositif d'affichage (18) mobile à la station d'acquisition d'images par ultrasons (46) et pour permettre de contourner le second système d'exploitation et de faire fonctionner le dispositif d'affichage mobile (18) par le premier système d'exploitation.

12. Système d'imagerie par ultrasons (10) selon la revendication 1, dans lequel le système d'imagerie par ultrasons (10) est en outre configuré de telle sorte qu'au moins une partie du premier système d'exploitation est copiée dans la seconde unité de mémoire (44) du dispositif d'affichage (18) mobile sous la commande du premier système d'exploitation de l'ensemble d'acquisition d'images (46).
